# EUROPEAN PATENT SPECIFICATION

(11) **EP 2 981 288 B1**
(45) Date of publication and mention of the grant of the patent: **02.09.2020**
(21) Application number: 14779382.2
(22) Date of filing: 03.04.2014
(51) Int. Cl.: A61K 39/395, A61K 35/12, A61P 31/18, A61P 31/12

(54) **EXPRESSION OF HIV INHIBITORS BY MESENCHYMAL STEM CELLS**
EXPRESSION VON HIV-INHIBITOREN DURCH MESENCHYMALE STAMMZELLEN
EXPRESSION D'INHIBITEURS DU VIH PAR DES CELLULES SOUCHES MÉSENCHYMATEUSES

(30) Priority: 03.04.2013 US 201361808097 P
(43) Date of publication of application: 10.02.2016
(73) Proprietor: Tulane University, New Orleans, LA 70112-2632 (US)
(72) Inventor: BRAUN, Stephen, E., New Orleans, LA 70112 (US); MONDAL, Debasis, New Orleans, LA 70112 (US); BUNNEL, Bruce, A., New Orleans, LA 70112 (US); LEE, Narae, Berkeley, CA 94720 (US)
(74) Representative: Barker Brettell LLP
(86) International application number: PCT/US2014/032832
(87) International publication number: WO 2014/165677

(56) References cited:
- WO-A1-2009/087110
- WO-A2-03/025167
- WO-A2-2010/111522
- US-A1- 2003 059 412
- US-A1- 2004 009 188
- US-A1- 2006 241 027
- US-A1- 2011 027 240
- LEE NARAE ET AL: "The Potential Therapeutic Role of Secreted Antiviral Entry Inhibitory (SAVE) Peptides Expressed by Transduced MSCs To Block Viral Replication", MOLECULAR THERAPY, NATURE PUBLISHING GROUP, GB, vol. 21, no. Suppl.1, 18 May 2013 (2013-05-18), page S76, XP009191022, ISSN: 1525-0016
- GRANT D. TROBRIDGE ET AL: "Protection of Stem Cell-Derived Lymphocytes in a Primate AIDS Gene Therapy Model after In Vivo Selection", PLOS ONE, vol. 4, no. 11, 2 November 2009 (2009-11-02), page e7693, XP055270304, DOI: 10.1371/journal.pone.0007693
- R C ZAHN ET AL: "Efficient entry inhibition of human and nonhuman primate immunodeficiency virus by cell surface-expressed gp41-derived peptides", GENE THERAPY, vol. 15, no. 17, 1 May 2008 (2008-05-01), pages 1210-1222, XP055103215, ISSN: 0969-7128, DOI: 10.1038/gt.2008.73
- JAN VAN LUNZEN ET AL: "Transfer of Autologous Gene-modified T Cells in HIV-infected Patients with Advanced Immunodeficiency and Drug-resistant Virus", MOLECULAR THERAPY, NATURE PUBLISHING GROUP, GB, vol. 15, no. 5, 1 May 2007 (2007-05-01), pages 1024-1033, XP008092068, ISSN: 1525-0016
- JAKOB REISER ET AL: "Potential of mesenchymal stem cells in gene therapy approaches for inherited and acquired diseases", EXPERT OPINION ON BIOLOGICAL THERAPY, vol. 5, no. 12, 30 December 2005 (2005-12-30), pages 1571-1584, XP055289490, ASHLEY, LONDON; GB ISSN: 1471-2598, DOI: 10.1517/14712598.5.12.1571
- KIEM H P ET AL: "Hematopoietic-Stem-Cell-Based Gene Therapy for HIV Disease", CELL STEM CELL, ELSEVIER, CELL PRESS, AMSTERDAM, NL, vol. 10, no. 2, 3 February 2012 (2012-02-03), pages 137-147, XP002731081, ISSN: 1934-5909, DOI: 10.1016/J.STEM.2011.12.015 [retrieved on 2012-02-02]
- SCOTT G KITCHEN ET AL: "Stem cell-based anti-HIV gene therapy", VIROLOGY, ELSEVIER, AMSTERDAM, NL, vol. 411, no. 2, 19 December 2010 (2010-12-19), pages 260-272, XP028185460, ISSN: 0042-6822, DOI: 10.1016/J.VIROL.2010.12.039 [retrieved on 2010-12-24]
- KIMPEL JANINE ET AL.: 'Survival of the fittest: positive selection of CD 4+ T cells expressing a membrane-bound fusion inhibitor following HIV-1 infection' PLOS ONE vol. 5, no. 8, 2010, page E12357, XP055101818
- EGELHOFER, MARC ET AL.: 'Inhibition of human immunodeficiency virus type 1 entry in cells expressing gp41-derived peptides' JOURNAL OF VIROLOGY vol. 78, no. 2, 2004, pages 568 - 575, XP009029114
- Alejandro B Balazs ET AL: "Antibody gene transfer for HIV immunoprophylaxis", Nature immunology, 1 January 2013 (2013-01-01), page 1, XP055471884, United States DOI: 10.1038/ni.2480 Retrieved from the Internet: URL:https://www.nature.com/articles/ni.248 0.pdf
- CHEN W ET AL: "Bifunctional fusion proteins of the human engineered antibody domain m36 with human soluble CD4 are potent inhibitors of diverse HIV-1 isolates", ANTIVIRAL RESEARCH, ELSEVIER BV, NL, vol. 88, no. 1, 1 October 2010 (2010-10-01), pages 107-115, XP027317853, ISSN: 0166-3542 [retrieved on 2010-08-13]
- LU LU ET AL: "A bivalent recombinant protein inactivates HIV-1 by targeting the gp41 prehairpin fusion intermediate induced by CD4 D1D2 domains", RETROVIROLOGY, BIOMED CENTRAL LTD., LONDON, GB, vol. 9, no. 1, 7 December 2012 (2012-12-07), page 104, XP021129366, ISSN: 1742-4690, DOI: 10.1186/1742-4690-9-104
- Suzanne L. Tomchuck ET AL: "Mesenchymal stem cells as a novel vaccine platform", Frontiers in Cellular and Infection Microbiology, vol. 2, 16 November 2002 (2002-11-16), pages 1-8, XP055286040, DOI: 10.3389/fcimb.2012.00140

### SEQUENCE LISTING

<110> TULANE UNIVERSITY
<120> EXPRESSION OF HIV INHIBITORS BY MESENCHYMAL STEM CELLS
<130> Tu504
<150> US 61/808,097
   <151> 2013-04-03
<160> 2
<170> PatentIn version 3.5
<210> 1
   <211> 45
   <212> PRT
   <213> Artificial Sequence
<220>
   <223> Synthetic peptide
<400> 1
   Trp Met Glu Trp Asp Arg Glu Ile Asn Asn Tyr Thr Ser Leu Ile His
   1 5 10 15
   Ser Leu Ile Glu Glu Ser Gln Asn Gln Gln Glu Lys Glu Gln Glu Leu
   20 25 30
   Leu Glu Leu Asp Lys Trp Ala Ser Leu Trp Asn Trp Phe
   35 40 45
<210> 2
   <211> 36
   <212> PRT
   <213> Artificial Sesquence
<220>
   <223> Synthetic peptide
<400> 2
   Tyr Thr Ser Leu Ile His Ser Leu Ile Glu Glu Ser Gln Asn Gln Gln
   1 5 10 15
   Glu Lys Asn Glu Gln Glu Leu Leu Glu Leu Asp Lys Trp Ala Ser Leu
   20 25 30
   Trp Asn Trp Phe
   35

## Description

### PRIOR RELATED APPLICATIONS

This disclosure claims priority to US 61/808097, filed on April 3, 2013.

### FEDERALLY SPONSORED RESEARCH STATEMENT

Not applicable.

### REFERENCE TO MICROFICHE APPENDIX

Not applicable.

### FIELD OF THE DISCLOSURE

The present disclosure relates generally to methods of treating human immunodeficiency virus (HIV), and specifically, to the use of transduced mesenchymal stem cells to treat HIV.

### BACKGROUND OF THE DISCLOSURE

Human immunodeficiency virus (HIV) is a slowly replicating retrovirus that leads to progressive failure of the immune system in humans, thus opening the door for life-threatening opportunistic infections and cancers to thrive. Infection with HIV results in CD4⁺ T cells depletion through a number of mechanisms including: apoptosis of uninfected bystander cells; direct viral killing of infected cells; and, killing of infected CD4+ T cells by CD8 cytotoxic lymphocytes that recognize infected cells. The loss of the CD4⁺ T cells causes Acquired Immune Deficiency Syndrome (AIDS). The loss of immune function from HIV and AIDS has led to the death of over 25 million people from AIDS worldwide.

Many therapies exist to manage but not cure HIV. Antiretroviral therapies (ART), for example, control viremia, a condition where viruses enter the bloodstream and have access to the rest of the body. The available anti-HIV drugs target different phases of the HIV life cycle, wherein each drug is classified according to the phase it inhibits. However, a standard ART consists of at least three antiretroviral drugs used in combination such that multiple phases of the HIV life cycle are inhibited and replication is suppressed as much as possible.

While showing promise in HIV and AIDS treatment, ART does have its drawbacks. Over 30 different types of drugs are available for ART, but only a few combinations may work for a given patient. A patient may have to cycle through multiple combinations and dosage levels before finding one that works. Multiple pills must be taken one or more times a day on a specific schedule to decrease a patient's viral load. Any deviation from the treatment schedule can result in mutations of the HIV/AIDS strain that may be resistant to the current drug combination. Furthermore, these medications may cause side effects, such as insulin resistance or lipid abnormalities, which also require additional medication in combination with the ART. Thus, the ART drug regimens are complex and expensive, require life-long intervention with potential side effects.

Like most viruses, HIV has a viral envelope. Embedded in HIV's viral envelope is a protein known as Env, which consists of a cap made of three molecules called glycoprotein (gp) 120, and a stem consisting of three gp41 molecules that anchor the virus structure into the viral envelope. Because this glycoprotein complex enables the virus to attach to and fuse with target cells to initiate infection, the glycoproteins are targeted by treatments and vaccines.

Enfuvirtide (fuzeon, or T-20) is an FDA-approved drug in a new class of fusion inhibitor that disrupts the HIV-1 molecular machinery at the final stage of fusion with the target cell. Enfuvirtide targets the gp41 glycoprotein during fusion. Specifically, gp41 undergoes conformational changes that allow fusion of HIV-1 to target cells. Enfuvirtide binds to gp41, thus preventing the creation of an entry pore for the virus, keeping it out of the cell.

However, this injectable drug has significant side effects and drug delivery issues as most patients develop some local injection site reaction. Furthermore, enfuvirtide has a short half-life, which requires twice daily administration, thus antagonizing the local injection site reaction. The subcutaneous application of enfuvirtide is also a distinct disadvantage in patients who are already burdened by complex oral therapy. Other alternative treatments include that of WO 2009/087110 A1, which discloses a HIV gene therapy. The gene therapy involves providing a patient with a nucleic acid for the expression of *in vivo* secretable peptides that prevent entry of the virus into the cell. The invention also relates to nucleic acids and vectors containing the same that code for a fusion protein which contains two HIV entry inhibitory peptides which are connected by a cleavable linker, wherein the peptides are preferably derived from the second (C) heptad repeat of the gp41 protein of HIV. Also, Lee Narae et al., 2013 (Molecular Therapy, Nature Publishing Group, GB, vol 21, Suppl. 1: page S76) disclose a potential therapeutic role of secreted Antiviral Inhibitory (SAVE) peptides expressed by transduced MSCs to block viral replication.

Nevertheless, there exist a need for novel therapies that are less complex and more accommodating. Ideally, the new therapies will have limited side effects and a longer half-life.

### SUMMARY OF THE DISCLOSURE

According to one aspect of the invention there is provided a pharmaceutical composition comprising mesenchymal stem cells (MSCs), wherein the MSCs have been transduced with a viral vector encoding a human immunodeficiency virus (HIV) antiviral fusion inhibitor, wherein said MSCs express and secrete the HIV antiviral fusion inhibitor, and wherein the HIV antiviral fusion inhibitor is an antibody. The disclosure also relates to a method of inhibiting HIV infection using transduced MSC expressing these inhibitors. According to another aspect of the invention, there is provided a composition comprising transfected mesenchymal stem cells (MSC) transduced with a viral vector encoding a HIV neutralising antibody, wherein said MSC express and secrete the HIV neutralising antibody, for use in treating human immunodeficiency virus (HIV) in a subject.

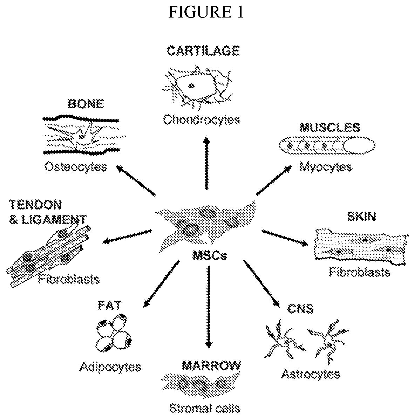

Multipotent stem cells, such as mesenchymal stem cells (MSCs), are stem cells capable of differentiating into cells specific to tissues and organs containing these cells and are involved in fetal, neonatal and adult periods of growth and development, but also in the maintenance of homeostasis of adult tissue and the functioning of inducing regeneration upon tissue damage. The variety of cell types that MSCs can differentiate into is illustrated in FIG. 1.

MSCs have been proposed for the treatment of numerous illnesses in part because these cells have been shown to migrate to sites of inflammation. Unlike most other human adult stem cells, MSC can be obtained in quantities appropriate for clinical applications, making them good candidates for use in tissue repair. Furthermore, they also have antiproliferative, immunomodulatory and anti-inflammatory effects and are low immunogenic. Although the mechanism underlying the immunosuppressive effects of MSCs has not been clearly defined, their immunosuppressive properties have already been exploited in the clinical setting. Therefore, MSCs have implications for treatment of allograft rejection, graft- versus-host disease, rheumatoid arthritis, autoimmune inflammatory bowel disease and other disorders in which immunomodulation and tissue repair are required.

In addition to tissue repair, MSCs have also been used in certain virus treatments. WO2010053350 describes the use of MSCs for inhibition of a hepatitis viral infection. Here, cells replicating the virus are contacted with either a liquid containing MSCs or with an exudate of the MSCs. However, MSCs have never been used to express inhibitors of viruses such as HIV.

In the present disclosure, it was determined that MSCs can be used as "trojan bioreactors" to carry and express HIV inhibitors at local sites of infection. The MSCs were transduced with retroviral and lentiviral vectors packaged with antiviral fusion inhibitors of the HIV infection. The transduced MSCs were able to express the inhibitors and maintain differentiation abilities. In addition to inhibiting HIV fusion, these transduced MSC are expected to reduce the general immune activation frequently observed in patients with HIV infection as has been shown by MSC treatment in other disease indications.

In one embodiment, the present disclosure provides a pharmaceutical composition comprising transduced MSCs expressing certain inhibitors to treat HIV as defined by claim 1. Specifically, HIV anti-viral fusion inhibitors are incorporated into a viral vector such as a retrovirus or a lentivirus. These viral vectors vehicles are introduced into the MSCs, wherein the cells under go transduction. The transduced MSC are then able to express the HIV inhibitors while retaining important characteristics such as the capacity for osteogenic, adipogenic, and chondrogenic differentiation,

The disclosure also relates to a method of treating human immunodeficiency virus by administering mesenchymal stems cells expressing anti-viral fusion inhibitors of HIV infection is disclosed. The MSCs will provide long-term, continuous expression of the inhibitors without the side effects associated with currently available injectable HIV inhibitors, such as injection-site irritation and difficulties in handling.

The disclosure also relates to a method of inhibiting HIV infection by administering conditioned medium from transduced MSC expressing anti-viral fusion inhibitors.

Additionally, the above methods can be combined with an anti-HIV drug treatment. The MSCs can be administered before, during or after the antiviral drugs are administered. Preferably, the MSCs are administered after the antiviral drugs.

The transduced MSCs may be administered directly to a subject. The MSCs or conditioned media may be mixed with an appropriate pharmaceutical carrier for administration. Preferred routes of administration are intravenous, subcutaneous and intraperitoneal injections.

Multiple HIV anti-viral fusion inhibitors can be used with the present composition and methods. Peptide inhibitors that block different points in the HIV replication and fusion mechanism by targeting CCR5, CD4-binding, gp41 and envelope can be used in the above embodiments. In addition to, or in place of, peptide inhibitors, neutralizing antibodies can be packaged into the vectors. Neutralizing antibodies defend a cell from an antigen or infectious body by inhibiting or neutralizing any effect it has biologically.

Exemplary peptide inhibitors are C46 peptides, which are derived from the HR2 gene of gp41. Secretable antiviral entry inhibitory (SAVE) peptides for C46 such as those in US20110027240 can also be used. For instance, a customized C46 peptide:
WMEWDREINNYTSLIHSLIEESQNQQEKEQELLELDKWASLWNWF (SEQ NO.1) was used in some of the present disclosed methods. T-20: YTSLIHSLIEESQNQQEKNEQELLELDKWASLWNWF (SEQ No. 2), provided through the NIH AIDS Reagent Program, division of AIDS, NIAID, NIH: T-20, fusion Inhibitor from AIDS, NIAID, is another possible peptide inhibitor. Such SAVE peptides are known to disrupt the fusion mechanism by blocking the gp41 viral envelope protein. Other peptide inhibitors of HIV replication, in addition to the C46 SAVE peptides, would be acceptable in the presently disclosed composition and method.

The sequence of the antiviral fusion inhibitors are incorporated into retroviral and lentiviral vectors before being transduced in the MSCs. Success of the transduction is confirmed by analyzing the MSCs using commonly acceptable practices to confirm expression of the peptide inhibitors. Once expression is confirmed, the MSCs can be injected into a person who has HIV, who has been exposed to HIV, or who is at risk for exposure to HIV.

The inhibitors can be packaged into the retroviral and lentiviral vectors using any known method in the art, typically, using purchased kits. Invitrogen offers lentiviral packaging kits, as does InvivoGen. Additionally, markers such as green fluorescence protein can also be packaged to facilitate confirmation of expression and determine transduction efficiency.

The MSCs for the present methods can be allogeneic or autologous, depending on the stage of infection and degree of immune deficiency. All acceptable methods of extraction of MSC can be used in the present methods. Preferably, MSCs derived from adipose tissue are utilized due to the large number of cells per volume of sample. Furthermore, adipose tissue is much easier to access than other sources such as bone marrow, which is particularly important for immune-compromised subjects. However, because MSCs can be expanded in culture before being transduced, other sources of MSCs, such as bone marrow, can be utilized.

The described composition and treatment methods can be applied to animals, preferably mammals, and most preferably, to humans.

In the context of the disclosure, "HIV" includes HIV-1 and HIV-2 types. Further included are all natural HIV strains (quasi-species or isolates). In this respect, it is understood that HIV strains include all known HIV strains as listed in the Los Alamos National Laboratory HIV Sequence Database.

As used herein, "mesenchymal stem cells" means any cells that meet the criteria set forth by The Mesenchymal and Tissue Stem Cell Committee of the International Society for Cellular Therapy (ISCT). Under this criteria, MSCs: 1) are plastic-adherent when maintained under standard culture conditions (especially in the presence of human or fetal bovine serum); 2) have the capacity for osteogenic, adipogenic, and chondrogenic differentiation; 3) express CD73, CD90, and CD105; and 4) lack expression of the hematopoietic lineage markers c-kit, CD14, CD11b, CD34, CD45, CD19, CD79, and human leukocyte antigen-DR.

The term "autologous" as used herein shall be taken to mean from the same individual.

The term "allogeneic" as used herein shall be taken to mean from an individual separate from the individual being treated.

The use of the word "a" or "an" when used in conjunction with the term "comprising" in the claims or the specification means one or more than one, unless the context dictates otherwise.

The term "about" means the stated value plus or minus the margin of error of measurement or plus or minus 10% if no method of measurement is indicated.

The use of the term "or" in the claims is used to mean "and/or" unless explicitly indicated to refer to alternatives only or if the alternatives are mutually exclusive.

The terms "comprise", "have", "include" and "contain" (and their variants) are open-ended linking verbs and allow the addition of other elements when used in a claim.

The phrase "consisting of' is closed, and excludes all additional elements.

The phrase "consisting essentially of" excludes additional material elements, but allows the inclusions of non-material elements that do not substantially change the nature of the disclosure.

The following abbreviations are used herein:

| ABBREVIATION | TERM |
|---|---|
| ART | Antiretroviral therapies |
| MSC | Mesenchymal Stem Cells |
| GFP | Green fluorescent protein |
| FACs | Fluorescence-activated cell sorting |
| Rh | Rhesus |
| BMS | Bone marrow MSCs |
| HIV | Human immunodeficiency virus |

### BRIEF DESCRIPTION OF THE DRAWINGS

FIG. 1. Illustration of potential cell types upon MSC differentiation.
FIG. 2 shows a diagram of the HIV fusion mechanism. From Drugs Fut. 1999, 24(12): 1355.
FIG. 3 shows the design of a number of vectors.
FIG. 4 shows a determination of transduction level using GFP+ cells.
FIG. 5 shows GFP+ levels by flow cytometry.
FIG. 6 shows confirmation of a transduced RhBMS Differentiation Assay.
FIG. 7A-B display western blots of MSCs tranduced with various HIV peptide inhibitors packaged in virus vectors, wherein RhBMS cells are in 7A and 293T cells are in 7B.
FIG. 8 displays a single round infection/inhibition assay in CEMx174 cells using conditioned media from MSC transduced with different inhibitors.

### DETAILED DESCRIPTION

The disclosure describes novel transduced mesenchymal stems cells (MSC) that express HIV virus-cell fusion inhibitors and a method of use to treat HIV. Specifically, mesenchymal stems cells (MSC) are transduced with one or more retroviral and/or lentiviral vectors expressing antiviral fusion inhibitors of HIV is disclosed. The transduced MSCs express the inhibitors without losing important MSC characteristics such as the ability to differentiate.

Methods of using the novel composition can comprise treating an animal, and preferably a human, suffering from an HIV infection wherein a peptide inhibitor or neutralizing antibody is incorporated into a viral or nonviral vector, which is then transduced into a MSC such that the MSC can express the inhibitor. The transduced MSCs are then administered to a subject alone or in combination with anti-retroviral therapies. The MSCs can be autologous or allogenic and taken from any source including bone marrow or adipose tissue. Preferred viral vectors are lentivirus, retrovirus, or any combination thereof. Exemplary inhibitors include one or more of secreted antiviral entry inhibitory (SAVE) peptides, C46, maC46 or neutralizing antibodies. The MSCs can be administered intravenously, subcutaneously, or intraperitonealy.

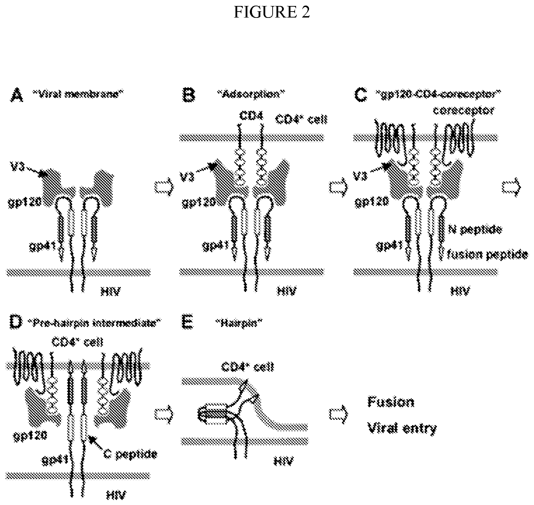

FIG. 2 displays the fusion mechanism by which HIV infects new cells. HIV fusion with a target cell starts with the attachment of gp120 to the N-terminal domain of the CD4+ receptors (B) Once attached, gp120 undergoes a conformational change that unmasks the binding site for the cell co-receptor (C). Once the virus is bound, a conformational change exposes the N-terminal hydrophobic region of gp41 to the CD4+ cell membrane and becomes the "pre-hairpin intermediate". Gp41 undergoes a conformation change (D), also referred to as a coiled-coil bundle, to bring the virus and cell membranes together, mediating fusion of virus and cell (E).

In one aspect of the present disclosure, to inhibit the fusion of HIV, MSCs are transduced with HIV peptide inhibitors. Many HIV peptide inhibitors exist including maC46 and C46 peptides as well as neutralizing antibodies. The HIV entry inhibitor maC46 is a membrane-anchored peptide derived from the second heptad repeat of the HIV-1 transmembrane glycoprotein gp41. C46 peptides are members of the new fusion inhibitor class of antiretroviral drugs that efficiently block infection of new cells by interfering with the function of gp41. The C46 peptides can be membrane-bound or secreted.

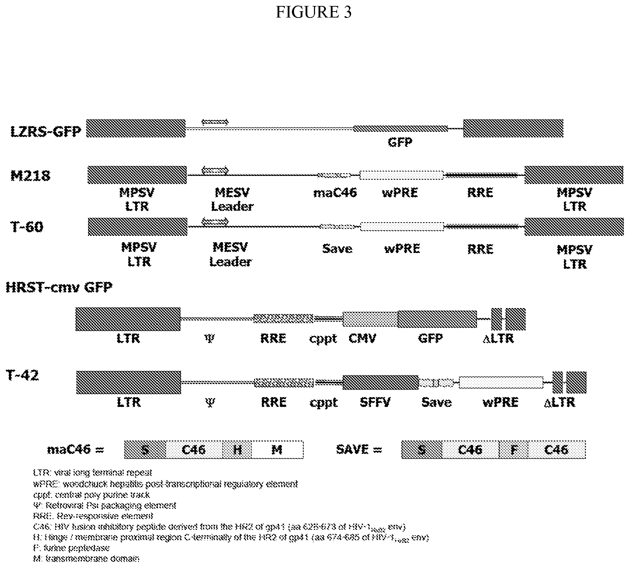

The peptide inhibitors are incorporated into retroviral or lentiviral vectors, which act as gene delivery vehicles for transducing the MSCs with the peptide inhibitors. Exemplary vectors include murine leukemia (MLV) and self-inactivating lentiviral vectors (HSRT), however other viral or non-viral vectors systems would also be applicable. FIG. 3 displays a few of the virus vectors that have incorporated the peptide inhibitors, particularly the maC46 and SAVE peptide sequences. The vectors can also contain a fluorescence marker, such as GFP, to aid in monitoring the success of the transduction.

After being transduced, the MSC can be analyzed to confirm expression of the peptide inhibitors using fluorescent microscopy, PCR, differentiation assays and other commonly used techniques.

The present disclosure is exemplified with respect to the examples and figures below. However, this is exemplary only, and the disclosure can be broadly applied to incorporate any HIV fusion inhibitor for all phases of the HIV life cycle and can be used for any animal. The following experiments and examples are intended to be illustrative only, and not unduly limit the scope of the appended claims.

### REFERENCE EXAMPLE 1

Gene transfer and expression was performed by the following steps:

**Cells:** Human Bone Marrow stem cells (HuMSCs), Rhesus Bone Marrow Stem cells (RhMSCs), and Rhesus Adipose stem cells (RhACSs) were plated at 10,000/cm² in alpha-MEM/20% FBS with no antibiotics the day before transduction.

For transduction, the plated cells were exposed to various MOI of the control vectors, such as LZRS-GFP (19) or HRST-cmvGFP (38), or the therapeutic vectors, such as T42 (unknown), T60 (22), and M218 (unknown), containing the C46 peptide sequences shown in FIG. 3. Then, the plates were centrifuged at 1000 rpm for 1-2 hours. After which, the MSCs were cultured at 37°C and 5% CO₂ for two days. After Day 2, cells were returned to standard culture conditions, wherein the cells were re-suspended in complete conditioned media and filter through a 70 mm cell strainer.

**Expression Confirmation:** Fluorescent microscopy and flow cyometry were used to assess the expression of the C46 peptide using GFP. Real time PCR was also performed.

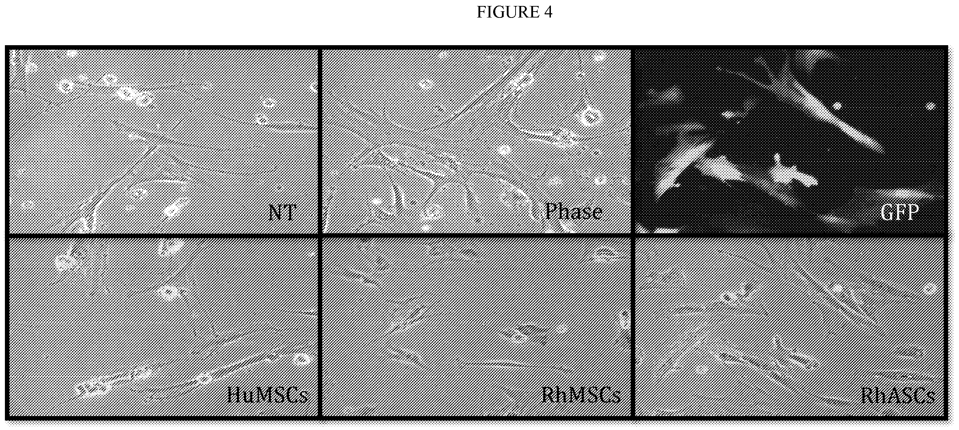

As shown in FIG. 4, cells were observed under fluorescent microscope to confirm transduction and expression of GFP in transduced cells. The upper panels are, from left, non-transduced (NT) MSC, rhASC with phase contrast, and GFP expression overlayed onto the phase contract image. Lower panels show overlay of GFP expression in HuMSCs, RhMSCs, and RhACSs transduced with HRST-cmvGFP. As seen, the GFP is readily expressed in the human and rhesus stem cells.

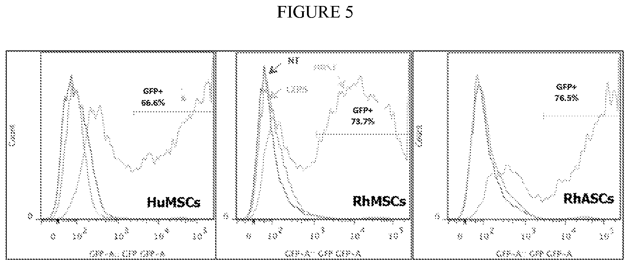

FIG. 5 displays the flow cytometry Fluoresence-activated cell sorting (FACs) results for HuMSCs, RhMSCs, and RhACSs. For this analysis, cells were trypsinized and washed with 1% mouse serum. After the wash, cells were fixed with 4% paraformaldehyde and analyzed for GFP expression by flow cytometry. The NT control population was set as background fluorescence.

The HRST-GFP showed the highest expression levels in all three populations by FACs analysis. The LZRS-transduced MSC showed slightly elevated levels of GFP expression in all three MSCs populations.

Gene transfer and expression was detectable up to 69% in MSCs by fluorescent microcopy and flow cytometry.

The real time PCR results for the vector backbone, shown below, detected between 5-25% of the human MSCs, rhesus MSCs, and HEK 293 cells contained the Psi packaging element in T60 and M218. Both T60 and M218 are murine leukemia virus based vectors.

| Cell type | C46 Vector | |
|---|---|---|
| | T60 (%) | M218 (%) |
| Hu MSC | 3.9 | 6.3 |
| Rh MSC | 12.5 | 25.5 |
| Rhs ASC | 10.6 | 14.7 |
| 293T | 10.0 | 13.3 |

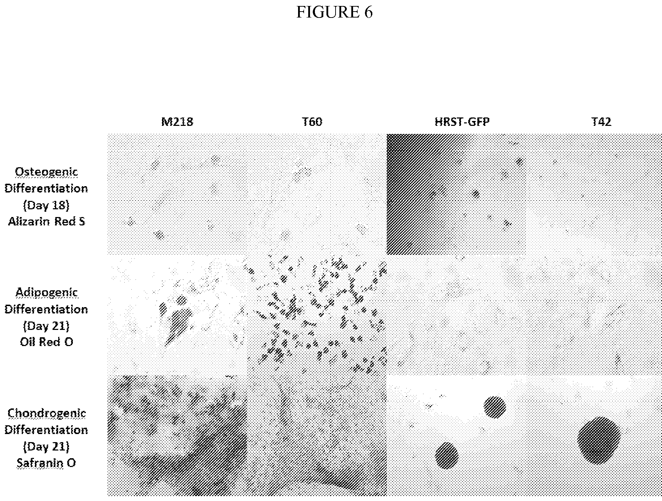

Differention assays were also performed to confirm transduced RhMSCs to test that the inhibitors do not disturb MSCs differentiation and results are shown in FIG. 6. The osteogenic, adipogenic, and chrondrogenic differentiation abilities of the MSCs were taken at 21 days for the M218, T60, HRST-GFP and T42 vector. As seen in FIG. 6, the MSCs retained their ability to differentiate into multiple cells, regardless of the vector or peptide inhibitor used. This shows that MSCs have not lost their differentiation abilities after being transduced.

**Results:** The HIV1-based lentiviral vector HRST-cmvGFP was superior to LZRS-GFP in both the frequency of gene transfer and the level of expression. Furthermore, real-time PCR data showed a transduction rate of 5-25% in the MSCs for MLV C46 vectors.

### EXAMPLE 2

The above method was applied to a larger variety of cells.

**Cells:** Most cells were obtained from the NIH AIDS Reagent Program. The NIH AIDS Reagent Program is a worldwide resource for state-of the art HIV research materials, some of which are not yet commercially available. CEMx174 cells (NIH AIDS Reagent Program Cat no. 272), expressing CD4, T and B cell markers, were obtained because they are easily infected with HIV virus. Second T-cell line "PM-1" (NIH AIDS Reagent Program Cat no. 3038), permissive for growth of macrophage and T cell tropic viruses, a subclone of HuT78 (Human T cell lymphoma) expressing CD4, CXCR4, and CCR5, was obtained. All T-cell lines were cultured in Roswell Park Memorial Institute medium (RPMI) 1640 medium.

Human TZM-bl cell (NIH AIDS Reagent Program Cat no. 8129), previously designated JC53-bl (clone 13) is a HeLa cell line. The parental cell line (JC.53) stably expresses large amounts of CD4 and CCR5. The TZM-bl cell line was generated from JC.53 cells by introducing separate integrated copies of the luciferase and β-galactosidase genes under control of the HIV-1 promoter. All media were supplemented with 10% fetal bovine serum, 4 mM L-glutamine, 10,000 U/mL penicillin/streptomycin.

Rhesus macaque bone marrow/adipocyte derived mesenchymal stem cells were isolated and maintained in complete culture medium, 20% FBS MEM α (Minimum Essential Medium α), with l-glutamine, fetal bovine serum (Atlanta Biologicals Premium Select FBS), Penicillin G (10,000 units/mL), and streptomycin sulfate (10,000 µg/mL) in solution of 0.85% NaCl (Invitrogen/GIBCO).

Human embryonic kidney cell line 293T was obtained from the ATCC, and was maintained in Dulbecco's modified Eagle's medium (DMEM).

**Bone marrow and adipose derived mesenchymal stem cells isolation and culture:** Bone marrow-derived mesenchymal stem cells were isolated by discontinuous density gradient centrifugation following the Tulane Center for Stem Cell Research and Regenerative Medicine protocol. Briefly, LSM (Lymphocyte Separation Medium, ICN50494, MP Biomedicals, No.:0850494) one-step separation medium was underlayered beneath the bone marrow mixture. The sample was centrifuged at 1400 rpm (∼ 400 g) for 40 min. The low-density mononuclear cells were collect at the white interface and washed with fresh media. After the last centrifugation, the supernatant discard and a lysis buffer (ACK lysing buffer, Gibco, catalog no. 10492-01) was used to eliminate residual red blood cells.

The collected cells were plated at a concentration of ∼1-2 million cells in a 15 cm tissue culture dish and incubate at 37°C in 5% CO₂. The complete culture medium was changed the next day (24 hours later) and replace with fresh complete medium. When the plate was about 70-80% confluent (∼ 14 days), cells were trypsinized and frozen at P0 in Liquid N₂ until they were expand to PI or frozen as a pellet for DNA and protein analysis.

Adipose-derived stem cells were isolated according to the Tulane Center for Stem Cell Research and Regenerative Medicine protocol. Briefly, the adipose tissue was washed extensively with a 5 % Phosphate Buffer Solution (PBS 5% p/s) before being minced into small pieces using a sterile scalpel. The pieces were placed in a 15 cm dish with 0.075% Collagenase Type I (Gibco, catalog no. 17100-017) in PBS. Collagenased tissues were incubated for 30 min at 37°C at 5% CO₂. The liquid portion was removed and the plate was washed several times with PBS 2% p/s, and centrifuge to obtain a pellet. After lysing with ACK Lysing Buffer (Gibco, catalog no. 10492-01), the adipose-derived cells were washed and incubated for 10 min on ice to lyse any red cells. The cells were again washed with 20 ml PBS 2% p/s, wherein the supernatant was discard. The cell pellets were re-suspended in complete conditioned media and filter through a 70 mm cell strainer.

Collected cells were plated into the proper size culture dish depending on the size of cell pellet and incubate at 37°C at 5% CO₂. The media was changed the next day and replaced with fresh media. When the plate was about 70-80% confluent, aspirate the medium. Cells were frozen and stored in liquid N₂ until they used to expand to PI, and then were frozen a pellet for DNA and protein analysis.

**Peptide cloning and mesenchymal stem cells transduction:** The C46 peptide used in the present experiment was SEQ No. 1.

C46 peptides driven by Murine leukemia virus (MLV) and lentivirus (M218, T60, and T42) and control vectors (LZRS-GFP and HRST-cmv-GFP) were transformed using one shot stbl3 kit with competent cells (Invitrogen, Cat. No. C7373-03) followed by manufacture's procedure. After sitting overnight, the colonies were selected and a single colony was grown in LB medium plus ampicillin overnight. DNA mini prep was performed with PureLink® Genomic DNA Mini Kit (Invitrogen, Catalog No. 1820-01).

Transient transduction of 293T cells with MLV and lentiviral vectors were completed by the calcium phosphate precipitation method. The calcium phosphate transduction method is used for introducing DNA into mammalian cells and is based on forming a calcium phosphate-DNA precipitate. Calcium phosphate facilitates the binding of the DNA to the cell surface. DNA then enters the cell by endocytosis.

15µg of MLV vector plasmid was used for LZRS-GFP and LZRS-T60 and 2ug of LZRS-M218 was used for transfection of the Phoenix (amphotropic) packaging cell line, a cell line bases on HEK 293T cells. They constitutively express the MLV Gag/Pol and amphotropic envelope genes. Addition of the vector genome will generate replication defective MLV-based retroviral particles in the supernatant. For the HIV-based lentiviral vectors, 13ug of HRST-GFP or T42 vector DNA was transfected along with the HIV packaging plasmids [pHDM-Hgm2 (gag/pol), pRC/Rev, pHDM-Tat 1b, envelope HIV-1 JRFL or VSV-G (pHDM.G)] or packaging vector (M438).

Cell culture supernatants containing replication defective lentiviral particles were collected from 24 to 48 hours post-transduction, filtered (0.22 µm pore size) and stored at -80 °C until use.

**Transduction of mesenchymal stem cells with lentiviral and MLV vectors:** 1x10⁵ cells/well of MSCs were plated in 6 well-plate a day before transduction. A desired amount of viral supernatant was added into each well, such as 1 ml of non-concentrated viral supernatant or 100ul of concentrated viral supernatant. MSCs were transduced with MLV vector and lentiviral vector supernatants by spinoculation (1hr, 1200 rpm, 31°C) then incubated at 37°C in 5% CO₂.

At day 2, the viral supernatant was removed and fresh 20% FBS alpha-MEM with no antibiotics was added to the MSCs. This media was change with 20% FBS alpha- MEM with no antibiotics at day 4. At day 6-7, depending on cell density, cells were prepared for fluorescent microscopy, flow cytometry, and quantitative proviral integrations by real-time PCR. Transduction efficacies were determined 3 days after transduction by flow cytometry.

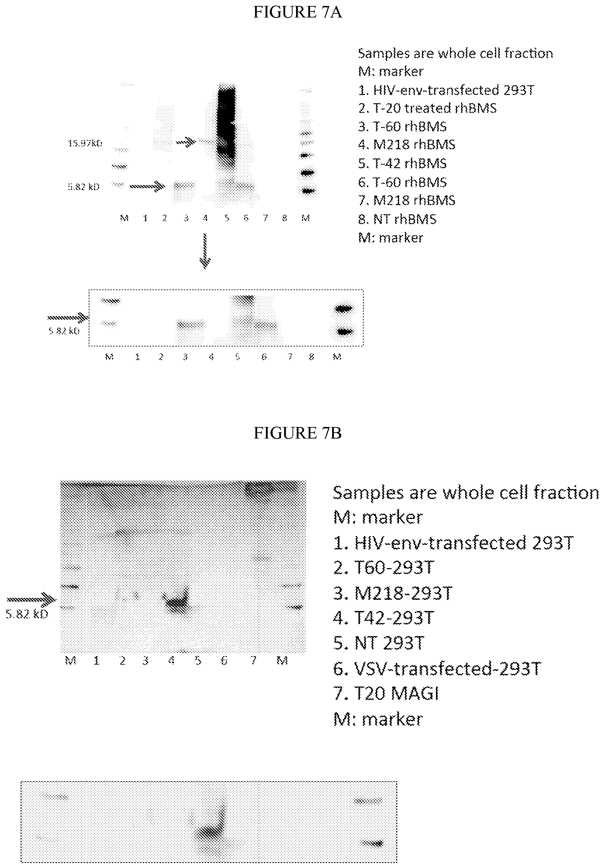

**Detection of secreted version of C peptides:** To determine the secreted C46 peptides in mesenchymal cell culture supernatants and cell lysates, a western blot analysis was performed and is shown in FIG. 7A and B. Human HIV-1 gp41 monoclonal antibody (2F5) was obtained from the NIH AIDS Reagent Program. 2F5 allowed the binding of the antibody to all available C peptides.

The secreted peptides were apparent at the 5.82 kD level. This shows that the peptide inhibitors are being secreted by the MSCs and can be available for HIV treatments.

**Preparation of HIV pseudotyped particles:** Day before transduction, 9x10⁶ cells of 293T cells were plated on 15cm culture dish for a total of 10 plates. Additional plates were prepared for VSV-G control. 80% of confluency was confirmed under microscopy on the day of transduction.

For the transient transfection, HRST-GFP was mixed with pHDM-Hgm2 (gag/pol), pRC/Rev, pHDM-Tat 1b, and envelope gene (either HIV-1 JRFL envelope or the VSV-G control envelope, pHDM.G). All the listed plasmid vectors were gift from Dr. Richard C. Mulligan, Harvard Gene Therapy Institute except JRFL envelope plasmid was provided by Dr. Dorothee von Laer from Innsbruck Medical University. 2.5M CaCl₂ and 2X HBS solution was added equal ratio. Final volume of 1ml of transduction mixture was applied into each culture dish. After 6 hours later, fresh 10% DMEM medium was replaced. Viral supernatant was collected 24-48hrs after transduction and filtered through 0.22um size and treated with 5X PEG-it virus precipitation solution (PEG-it™ Virus Precipitation Solution (5×), SBI, Cat. # LV810A-1/ LV825A-1) for 48-72hrs. Yellow beige colored pellets were confirmed and, following manufacture's protocol, the virus was concentrated by centrifuge at 1500g for 30min at 4°C. Virus pellets were collected and resuspended in PBS solution and stored at -80°C until used.

**Viral single round infection/inhibition assay of pseduotyped HIV-1 system by C46 transduced in MSCs:** In order to mimic the HIV infection system, HRST (CMV-GFP) was packaged with an HIV envelope, JRFL. The CD4+ cell line CEMx174 were treated with conditioned medium (CM) from the control (HRST-GFP, LZRS-GFP) or C46 (T-60, M218, and T-42) transduced MSC and infected with various MOI of concentrated pseudo HIV-1 viral-like particles produced by calcium phosphate method. After 48hrs, cells were harvested and fixed with 4% PFA. Samples were analyzed for GFP(+) by BD FAC Verse™ flow cytometry at Tulane National Primate Research Center. GFP (+) flow analysis was conducted by FlowJo version 10 program.

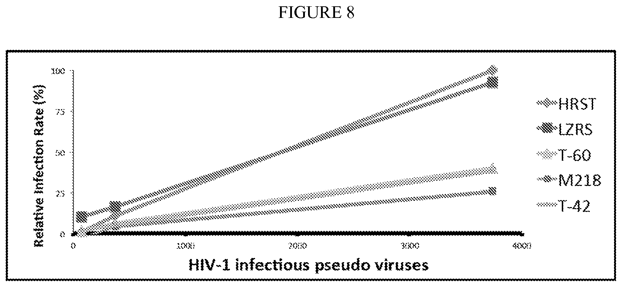

The resulting single round infection/inhibition assay is shown in FIG. 8. The CM from the control cells had the highest level of GFP expression (infection with the pseudoviral particles). Treatment with CM from the C46-transduced MSCs blocked infection. The level of GFP expression in the CEMx174 cells was graphed relative to the expression in the control population. Thus, the conditioned media from C46-transduced MSCs were able to block infection with HIV-1 pseudovirus in a single round inhibition assay.

**Mesenchymal stem cell differentiation assay:** To test that the inhibitors do not disturb MSCs differentiation, osteogenic, adipogenic, and chondrogenic differentiation assay with StemPro® Osteogenesis Differentiation Kit, Gibco A1007201, A1007001, and A1007101 was performed on LZRS-GFP, LZRS-T60, HRST-GFP, M218, T42, and non-transduced RhMSCs. Assay procedure was followed manufacture's protocol. Briefly, cell number to be seeded for assay was 5 × 10³ cells/cm² and 1 × 10⁴ cells/cm² into 12 wells for osteogenic and adipogenic differentiation respectively. 1.6 x 10⁷ viable cells/ml was plated by seeding 5-µl droplets of cell solution in the center of multi-well plate wells for classical stain for chondrogenic differentiation. Following around 21 days culture depends on cells growth, osteogenic, adipogenic, and chondrogenic differentiation were stained by alizarin red S, oil red o, and safranin o solution respectively. Stained cells were observed and taken pictures under bright field light microscope..

### REFERENCE EXAMPLE 3

The MSCs that expressed HIV inhibitor peptides in Experiments 1 and 2 can be combined with antiviral drugs *in vitro* to assess the synergetic effects. PMPA (Tenofovir disoproxil fumarate, TDF or tenofovir) and FTC (Emtriva or Emtricitabine) are exemplary antiviral drugs whose synergetic effects with the above MSCs can be measured in an *in vitro* cell culture model.

For instance, CEMx174 cells can be infected with various MOI of Simian-HIV 89.6p or HIV strains. Then, MSCs can be treated with Mitomycin C, which is used to generate mitotically inactive the MSC feeder cells. The CEMx174 cells can then be co-cultured with the treated MSC for up to 14 days with or without additional antiviral drugs (e.g. 2uM of PMPA and 1.5uM FTC or others). The HIV replication in the CEMx174 cells can be monitored using p27 flow cytometry or ELISA, or RT-PCR, or other method. Inhibition of viral replication will be quantified by limiting dilution assay.

Alternatively, CEMx174 cells can be treated with conditioned medium from the control (HRST-GFP, LZRS-GFP) or C46 (T-60, M218, or T-42) transduced MSCs and combined with the PMPA and FTC (or other standard HIV drug). Then, CEMx174 cells are exposed to the HIV viral-like particles. Inhibition of the HIV infection of the CEMx174 cells is measured by GFP expression.

Additionally, the order of treatment can be varied such that infected CEMx174 cells are treated with the transduced MSCs before the antiviral drugs or co-administered with the antiviral drugs.

### REFERENCE EXAMPLE 4

Animal model testing using the MSCs that expressed HIV inhibitor peptides in Experiments 1 and 2 alone or in combination with antiviral drugs can be performed with small and large animal models.

An exemplary small animal model is an immunodeficient mouse model. Table 1 lists many available mouse models and their advantages or disadvantages indicating that certain mouse models may be better candidates for animal testing.

| Table 1: Exemplary immunodeficient mouse model | | | |
|---|---|---|---|
| **Humanized Mouse Model** | **Cellular composition in reconstituted hu-mice** | **Advantages** | **Disadvantages** |
| Hu-PBL SCID | T and B cells | • Easy to generate | • No multilineage haematopoiesis |
| | | • Can immediately be used after transfer of PBLs | |
| | | | • Limited time fram for experiments |
| | | | • Strong activation of T-cells |
| | | | • Emergence of xeno-reactive T-cells (GVHD) |
| Thy/Liv SCID hu | T cells Single positive , double positive and double negative Thymocytes | • Organoid of fetal thymus/liver tissue with sustained T-cell lymphopoiesis | • Surgical skills needed |
| | | | Human fetal tissue needed |
| | | | • No multilineage hematopoiesis |
| | | • Valuable to study certain pathogenic aspects | |
| | | | • Lack of CCR5 expression on intrathymic T progenitor cells |
| Rag2 ^{-/-} 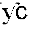 c^{-/-} | T, B cells, Monocytes, Macrophages, NK cells, and DCs | • Long term multilineage hematopoiesis | Delay between transplantation of human CD34+ cells and development of lymphoid systems of ∼15weeks |
| | | • Specific antibody response to recall antigens | |
| | | • Suited to study HIV pathogenesis, HIV latency, gene therapy and novel anti-HIV treatment approaches | |
| NOG or NSG | T, B cells, Monocytes, Macrophages, NK cells, and DCs | • Higher reconstitution levels as compared to Rag mice | Sensitive to irradiation |
| | | • Suited to study HIV pathogenesis, HIV treatment, gene therapy approaches | |
| NOD/SCID-hu BLT & NOD/SCID | T, B cells, Monocytes, Macrophages, | • Generation of adaptive immune responses | • Two stop procedure for generating BLT mice |
| | | • Suited to study HIV | • Surgical skills needed |
| 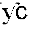 ^{-/-} (NSG) BLT | NK cells, and DCs | pathogenesis, HIV latency, gene therapy and novel anti-HIV treatment approaches | • Human fetal tissue needed |

The therapeutic potential of the stem cells in the humanized mouse models can be measured by analyzing the degree of human cell engraftment by flow cytometry of the peripheral blood mononuclear cells after they have been stained for panhuman markers such as CD45, CD3, CD4, CD8, CD14, and CD19.

Non-human primate models can be used for the large animal model testing. Such primate models would allow for the use of simian-HIV (S-HIV) infections. The S-HIV viral load in plasma of the animals can be measured using real time PCR. The therapeutic potential of the stem cells can be determined by analyzing the blood and tissue samples from infected primates for biodistribution of MSCs, toxicity and peptide measurement.

While certain novel features of this disclosure shown and described above are pointed out in the annexed claims, the disclosure is not intended to be limited to the details specified. No feature of the disclosure is critical or essential unless it is expressly stated as being "critical" or "essential."

Furthermore, the above experiments and examples are not intended to limit the scope of the disclosure composition or methods to certain cells or animals.

## Claims

1. A pharmaceutical composition comprising mesenchymal stem cells (MSCs), wherein the MSCs have been transduced with a viral vector encoding a human immunodeficiency virus (HIV) antiviral fusion inhibitor, wherein said MSCs express and secrete the HIV antiviral fusion inhibitor, and wherein the HIV antiviral fusion inhibitor is an antibody.

2. The pharmaceutical composition of claim 1, wherein said antibody is a neutralizing antibody.

3. A composition comprising transfected mesenchymal stem cells (MSC) transduced with a viral vector encoding a HIV neutralising antibody, wherein said MSC express and secrete the HIV neutralising antibody, for use in treating human immunodeficiency virus (HIV) in a subject.

4. The composition for the use of claim 3, wherein said MSCs are injected intravenously; and/or are injected subcutaneously; and/or are injected intraperitonealy.

5. The composition for the use of claim 3, wherein said subject is an animal.

6. The composition for the use of claim 3, wherein said subject is a human.

7. The composition for the use of claim 3, wherein the composition is administered with anti-retroviral therapies to said subject.

8. The composition for the use of claim 3, wherein the composition is for use in combination with antiviral drugs.

9. The composition for the use of claim 8, wherein one or more compositions from claims 1 or 2 are administered.

10. The composition for the use of claim 8, wherein the antiviral drugs are administered before the compositions in claims 1 or 2.

11. The composition for the use of claim 8, wherein the antiviral drugs are administered after the compositions in claims 1 or 2.

12. The composition for the use of claim 8, wherein the composition and antiviral drug are administered to an animal.

13. The composition for the use of claim 8, wherein the composition and antiviral drug are administered to a human.

## Patentansprüche

1. Pharmazeutische Zusammensetzung, umfassend mesenchymale Stammzellen (MSC), wobei die MSC mit einem Virusvektor transduziert wurden, der einen antiviralen HIV(Human Immunodeficiency Virus)-Fusionsinhibitor codiert, wobei die MSC den antiviralen HIV-Fusionsinhibitor exprimieren und wobei es sich bei dem antiviralen HIV-Fusionsinhibitor um einen Antikörper handelt.

2. Pharmazeutische Zusammensetzung nach Anspruch 1, wobei es sich bei dem Antikörper um einen neutralisierenden Antikörper handelt.

3. Zusammensetzung, umfassend transfizierte mesenchymale Stammzellen (MSC), die mit einem Virusvektor transduziert sind, der einen HIV neutralisierenden Antikörper codiert, wobei die MSC den HIV neutralisierenden Antikörper exprimieren und sezernieren, zur Verwendung beim Behandeln von HIV (Human Immunodeficiency Virus) bei einem Individuum.

4. Zusammensetzung zur Verwendung nach Anspruch 3, wobei die MSC intravenös injiziert werden; und/oder subkutan injiziert werden; und/oder intraperitoneal injiziert werden.

5. Zusammensetzung zur Verwendung nach Anspruch 3, wobei es sich bei dem Individuum um ein Tier handelt.

6. Zusammensetzung zur Verwendung nach Anspruch 3, wobei es sich bei dem Individuum um einen Menschen handelt.

7. Zusammensetzung zur Verwendung nach Anspruch 3, wobei die Zusammensetzung dem Individuum bei antiretroviralen Therapien verabreicht wird.

8. Zusammensetzung zur Verwendung nach Anspruch 3, wobei die Zusammensetzung zur Verwendung in Kombination mit antiviralen Arzneistoffen vorgesehen ist.

9. Zusammensetzung zur Verwendung nach Anspruch 3, wobei eine oder mehrere Zusammensetzungen aus Anspruch 1 oder 2 verabreicht werden.

10. Zusammensetzung zur Verwendung nach Anspruch 8, wobei die antiviralen Arzneistoffe vor den Zusammensetzungen unter Anspruch 1 oder 2 verabreicht werden.

11. Zusammensetzung zur Verwendung nach Anspruch 8, wobei die antiviralen Arzneistoffe nach den Zusammensetzungen unter Anspruch 1 oder 2 verabreicht werden.

12. Zusammensetzung zur Verwendung nach Anspruch 8, wobei die Zusammensetzung und der antivirale Arzneistoff einem Tier verabreicht werden.

13. Zusammensetzung zur Verwendung nach Anspruch 8, wobei die Zusammensetzung und der antivirale Arzneistoff einem Menschen verabreicht werden.

## Revendications

1. Composition pharmaceutique comprenant des cellules souches mésenchymateuses (CSM), les CSM ayant été transduites avec un vecteur viral codant pour un inhibiteur de fusion antiviral du virus de l'immunodéficience humaine (VIH), lesdites CSM exprimant et sécrétant l'inhibiteur de fusion antiviral de VIH, et l'inhibiteur de fusion antiviral de VIH étant un anticorps.

2. Composition pharmaceutique selon la revendication 1, ledit anticorps étant un anticorps neutralisant.

3. Composition comprenant des cellules souches mésenchymateuses (CSM) transfectées transduites avec un vecteur viral codant pour un anticorps neutralisant contre le VIH, lesdites CSM exprimant et sécrétant l'anticorps neutralisant contre le VIH, pour utilisation dans le traitement du virus de l'immunodéficience humaine (VIH) chez un sujet.

4. Composition pour l'utilisation selon la revendication 3, lesdites CSM étant injectées par voie intraveineuse ; et/ou étant injectées par voie sous-cutanée ; et/ou étant injectées par voie intrapéritonéale.

5. Composition pour l'utilisation selon la revendication 3, ledit sujet étant un animal.

6. Composition pour l'utilisation selon la revendication 3, ledit sujet étant un humain.

7. Composition pour l'utilisation selon la revendication 3, la composition étant administrée avec des thérapies antirétrovirales audit sujet.

8. Composition pour l'utilisation selon la revendication 3, la composition étant pour utilisation en combinaison avec des médicaments antiviraux.

9. Composition pour l'utilisation selon la revendication 8, une ou plusieurs compositions des revendications 1 ou 2 étant administrées.

10. Composition pour l'utilisation selon la revendication 8, les médicaments antiviraux étant administrés avant les compositions des revendications 1 ou 2.

11. Composition pour l'utilisation selon la revendication 8, les médicaments antiviraux étant administrés après les compositions des revendications 1 ou 2.

12. Composition pour l'utilisation selon la revendication 8, la composition et le médicament antiviral étant administrés à un animal.

13. Composition pour l'utilisation selon la revendication 8, la composition et le médicament antiviral étant administrés à un humain.
